# EUROPÄISCHE PATENTSCHRIFT

(11) **EP 3 173 034 B1**
(45) Veröffentlichungstag und Bekanntmachung des Hinweises auf die Patenterteilung: **17.10.2018**
(21) Anmeldenummer: 15196990.4
(22) Anmeldetag: 30.11.2015
(51) Int. Cl.: A61B 17/12

(54) **VORRICHTUNG FÜR DIE DURCHFÜHRUNG VON ORGANOKKLUSIONEN, INSBESONDERE EINER INTRAUTERINEN TRACHEALOKKLUSION BEI DER BEHANDLUNG EINER ANGEBORENEN FETALEN ZWERCHFELLHERNIE**
DEVICE FOR CARRYING OUT ORGAN OCCLUSIONS, IN PARTICULAR AN INTRA-UTERINE TRACHEAL OCCLUSION IN THE TREATMENT OF AN INNATE FETAL DIAPHRAGMATIC HERNIA
DISPOSITIF D'EXÉCUTION D'OCCLUSIONS ORGANIQUES, EN PARTICULIER D'UNE OCCLUSION TRACHÉALE INTRA-UTÉRINE LORS DU TRAITEMENT D'UNE HERNIE DIAPHRAGMATIQUE CONGÉNITALE

(43) Veröffentlichungstag der Anmeldung: 31.05.2017
(73) Patentinhaber: Universitätsklinikum Halle (Saale), 06120 Halle (Saale) (DE)
(72) Erfinder: TCHIRIKOV, Michael, 06126 Halle (Saale) (DE)
(74) Vertreter: Patentanwälte Dr. Keller, Schwertfeger Partnerschaft mbB

(56) Entgegenhaltungen:
- EP-A1- 1 974 685
- US-A- 3 452 749
- US-A- 5 792 179
- US-A1- 2004 254 625
- US-A1- 2009 024 228
- J A Deprest ET AL: "Tracheoscopic endoluminal plugging using an inflatable device in the fetal lamb model", European journal of obstetrics, gynecology, and reproductive biology, 1. Dezember 1998 (1998-12-01), Seiten 165-169, XP055255858, IRELAND Gefunden im Internet: URL:http://ac.els-cdn.com/S030121159800183 3/1-s2.0-S0301211598001833-main.pdf?_tid=4 c5fb3d8-e474-11e5-9c89-00000aacb361&acdnat =1457362565_81dcc78bf2413a61876fa260754d78 2c [gefunden am 2016-03-07]

## Beschreibung

Die vorliegende Erfindung betrifft eine Vorrichtung zur Verwendung bei Organokklusionen bei Menschen oder Säugetieren, insbesondere für die Durchführung einer intrauterinen Trachealokklusion bei Behandlung einer angeborenen fetalen Zwerchfellhernie.

Künstlich herbeigeführte Okklusionen von Organen bei Menschen oder Säugetieren sind für die Vorbeugung oder Behandlung einer Reihe von Erkrankungen bevorzugte Heilmethoden, was an die Entwicklung von Medizinprodukten besondere Herausforderungen stellt. Dies trifft insbesondere auf fetale Okklusionen zu, da hier erhebliche Behandlungsrisiken vorliegen, die sorgfältig gegenüber den üblichen Vorteilen abgewogen werden müssen. Die kongenitale Zwerchfell- oder Diaphragma-Hernie ist mit einer Häufigkeit von 1 bis 2 unter 5000 Schwangerschaften eine seltene und schwere Fehlbildung, die pränatal zu einer fetalen Lungenfehlentwicklung führt, was beispielsweise zur Verlagerung der Bauchorgane, vor allem der Leber, in der Thoraxhöhle führt (Skari H, et al., Congenital diaphragmatic hernia: a meta-analysis of mortality factors. J Pediatr Surg 2000;35:1187-1197; Rotter et al., Fetal lung and diaphragm development in congenital diaphragmatic hernia. Semin Perinatol 2005;195:1720-1728). Dagegen sind postnatale Defekte häufig einfach chirurgisch behandelbar.

Die betroffenen Lungen sind deutlich hypoplastisch und weisen eine verringerte Oberfläche für den Gasaustausch sowie eine geringe Anzahl an Alveolen auf, die zudem verdickte Wände haben (Areechon W et al.: Hypoplasia of lung with congenital diaphragmatic hernia; Br Med J 1963:5325:230-233). Dies führt aufgrund der resultierenden Lungenhypoplasie und Lungenhypertonie häufig zu einer schweren postnatalen Ateminsuffizienz, die trotz der zunehmenden Verfügbarkeit einer extrakorporalen Membranoxidierung (ECMO) in rund der Hälfte der Fälle zum Versterben der betroffenen Kinder führt (Beresford MW et al: Outcome of congenital diaphragmatic hernia. Pediatr Pulmonol 2000; 30: 249-256). Die Kinder, die überlebt haben, weisen zumeist eine bronchiopulmonale Dysplasie auf.

Die pränatale Diagnostik bei der Zwerchfellhernie umfasst beispielsweise eine Messung der Lungengröße mittels Ultraschall oder Magnetresonanz-Tomographie sowie eine Positionsbestimmung der Bauchorgane, beispielsweise der Leberposition. Abhängig von dem zu erwartenden postnatalen Verlauf muss daher aufgrund der recht hohen Mortalität und Morbidität überlegt werden, ob ein Schwangerschaftsabbruch oder eine pränatale Behandlung durchgeführt wird. Ziel einer solchen pränatalen Intervention ist nicht eine Korrektur des durch die Verlagerung der Bauchorgane bedingten anatomischen Defekts, sondern vielmehr eine Minimierung der Lungenfehlentwicklung sowie der Lungenhypoplasie. Ermöglicht wird dies durch die fetoskopische endoluminale Trachealokklusion (Deprest J et al: FETO Task Group: Fetoscopic tracheal occlusion (FETO) for severe congenital diaphragmatic hernia: evolution of a technique and preliminary results. Ultrasound Obstet Gynecol 2004; 24: 121-126). Eine vorübergehende Trachealokklusion stellt eine kongenitale Atemwegsobstruktion dar, bei der ein verstärktes Lungenwachstum auftritt. Dies beruht darauf, dass bei einem "Verschluss" der Trachea kein Abfluss der in der Lunge produzierten Flüssigkeit möglich ist. Dadurch kommt es zu einer vermehrten Dehnung der Lunge, die wiederum zur erhöhten Expression verschiedener Wachstumsfaktoren und damit zu einem gesteigerten Lungenwachstum führt (DiFiore JW et al: Experimental fetal tracheal ligation reverses the structural and physiological effects of pulmonary hypoplasia in congenital diaphragmatic hernia. J Pediatr Surg 1994; 29: 248-256; Deprest JA et al: Tracheoscopic endoluminal plugging using an inflatable device in the fetal lamb model. Eur J Obstet Gynecol Reprod Biol 1998; 81: 165-169).

Bei der fetoskopischen Therapie wird im Rahmen eines invasiven Eingriffes ein Fetoskop über die mütterliche Bauchdecke in die Gebärmutter und dort über den Mund des Kindes in die Trachea eingeführt (Deprest JA et al: Fetal surgery for congenital diaphragmatic hernia is back from never gone. Fetal Diagn Ther 2011; 29: 6-17). Anschließend wird ein Ballon bis vor die Carina vorgeschoben, mit Flüssigkeit entfaltet und platziert. Dafür kann auch der fetoskopische Kanal des Trokares unter der 3-D Ultraschallkontrolle benutzt werden (Tchirikov M. Successful tracheal occlusion using ultra-thin fetoscopic equipment combined with real-time 3D ultrasound. Eur Surg Res 2009;43(2):204-7).

Der Ballon verhindert einen Abfluss von Lungenflüssigkeit und wird in der Regel für einen Zeitraum von 4 bis 6 Wochen in der Trachea des Kindes belassen und anschließend auf gleiche Weise wieder entfernt. Bislang konnte man durch diese Behandlungsmethode die Überlebensrate von den zu erwartenden 10 bis 20 % auf ca. 50 % erhöhen, und es zeigte sich eine deutliche Reduktion der postnatalen Morbidität.

Nach wie vor schrecken die hohen Mortalität und Morbiditätsraten jedoch viele Mütter von der Anwendung einer fetoskopischen endoluminalen Trachealokklusion (FETO) ab, so dass diese einen Schwangerschaftsabbruch bevorzugen, denn die klassische Behandlung einer angeborenen Zwerchfellhernie durch eine Trachealokklusion mit einem Ballon ist risikobehaftet, was mehrere Ursachen hat. So ist zur Entfernung des intratrachealen Ballons ein zweiter fetal-chirurgischer Eingriff notwendig, der in der Regel zwischen der 34. und 35. Schwangerschaftswoche (SSW) liegt. Ferner bestehen die Risiken eines iatrogenen Blasensprungs sowie einer Frühgeburt. Der iatrogene Blasensprung stellt eine häufige Komplikation dar, die maßgeblich auch auf die zu verwendenden Geräte sowie Ballons und die damit verbundene Traumatisierung der amnialen Membran zurückzuführen ist. Ein weiteres Problem stellt sich mit der deutlich erschwerten Sicherung der kindlichen Atemwege bei liegendem Ballon im Fall einer ungeplanten Entbindung vor dem zweiten Eingriff (Harrison MR, Keller RL, Hawgood SB et al. A randomised trial of fetal endoscopic tracheal occlusion for severe fetal congenital diaphragmatic hernia. N Engl J Med 2003;349:1916-1924; Deprest J, Jani J, Gratacos E et al. Fetal intervention for congenital diaphragmatic hernia: the European experience. Semin Perinatol 2005;29:94-103). In diesem Fall stellt ein "EXIT-Verfahren ("es utero intrapartum treatment)" den modus operandi dar, bei dem zuerst bei Kaiserschnitt der Kopf des Kindes über eine Uterotomie entwickelt wird, anschließend eine Sicherung der Atemwege durch Entfernung des Ballons und Intubation des Kindes intrapartal erfolgt und erst anschließend der kindliche Rumpf entwickelt wird. Diese Prozedur ist jedoch mit erheblichen Risiken behaftet, was zumeist auf eine erschwerte Entfernung des Trachealballons sowie schwierige Intubationsbedingungen zurückzuführen ist. Wenn der Ballon aus der Trachea nach der Geburt nicht sofort entfernt wird, verstirbt der Neugeborene.

Mit ähnlichen Problemen der Ballonentfernung oder gar gänzlich fehlenden, nicht medikamentösen Behandlungsmethoden ist man auch bei anderen Indikationen, wie beispielsweise Nasenblutungen oder intrakorporalen Organblutungen, konfrontiert. So treten bei bestimmten Erkrankungen, beispielsweise bei Tumorerkrankungen, erhebliche organspezifische Blutungen auf, die nur mit erheblichem Aufwand stationär gestillt werden können. Durch die Einführung eines Ballonkörpers in die betreffende Organhöhle (beispielsweise in die blutende Nase oder in die Gebärmutter) ließen sich solche Organblutungen drastisch vermindern, beispielsweise indem der Ballonkörper Druck auf die betroffenen Blutgefäße des Organabschnitts ausübt und die Blutungen dadurch lindert oder gar gänzlich unterbindet.

Zwar sind zahlreiche Ballonimplantate bekannt, jedoch stellt die Entfernung des Ballons am Ende des Behandlungsabschnittes bei den im Zusammenhang mit Organokklusionen in Verbindung stehenden Indikationen ein erhebliches Problem für den Operateur und/oder den Patienten dar. So beschreibt beispielsweise die DE 103 02 241 A1 ein Ballonimplantat zur Okkludierung von Aneurismen. Der Ballon ist aus einem textilen Gewebe oder Gewirke hergestellt und enthält eine gerinnungsfördernde Flüssigkeit, beispielsweise Fibrin oder einen künstlich hergestellten Gewebekleber. Die EP 0 876 166 B1 beschreibt eine Biomaterialzusammensetzung zum Füllen und Abdecken von Hohlräumen oder Lumen eines Körpers, bei der eine Platzierung der Implantate unter Kontrolle erfolgt. Daneben gibt es eine Reihe von Ballonkathetern, wie beispielsweise in der DE 197 32 965 A1 zum Fixieren in Hohlorganen beschrieben, jedoch sind diese Medizinprodukte für Organokklusionen, insbesondere für einen diffizilen Eingriff im Rahmen einer intrauterinen Trachealokklusion bei der Behandlung einer angeborenen Zwerchfellhernie nicht geeignet. Die US 3,452,749 beschreibt ein Ballonkathetersystem, bei dem ein Faden vorgesehen ist, der mit dem Ballonmund verbunden ist. Dadurch soll ein Abtasten und Befüllen des Ballons nach dessen Platzierung in der Gebärmutter ermöglicht werden.

Vor diesem Hintergrund ist es Aufgabe der vorliegenden Erfindung, eine Vorrichtung bereitzustellen, welche sich zur Durchführung einer Organokklusion, insbesondere für die Durchführung einer intrauterinen Trachealokklusion bei der Behandlung einer angeborenen fetalen Zwerchfellhernie eignet und durch den Operateur oder den Patienten (d.h. den lebenden Fötus) selbst leicht entfernbar ist.

Gelöst wird diese Aufgabe durch eine Vorrichtung mit den Merkmalen des Anspruchs 1. Bevorzugte Ausführungsformen finden sich in den Unteransprüchen wieder.

Die erfindungsgemäße Vorrichtung ist zur Verwendung bei Organokklusionen bei Menschen oder Säugetieren bestimmt, insbesondere für die Durchführung einer intrauterinen Trachealokklusion im Rahmen der Behandlung einer angeborenen fetalen Zwerchfellhernie. Die Vorrichtung umfasst einen mit einem Fluid befüllbaren Ballonkörper, der aus einer Ballonhülle besteht, welche im Inneren ein Lumen zur Aufnahme des Fluids bildet, sowie einen Ballonmund. Der Ballonkörper ist erfindungsgemäß mit einem flexiblen länglichen Verlängerungselement verbunden. Vorzugsweise wird als Fluid für eine intrauterine Anwendung eine physiologische Lösung verwendet, beispielsweise eine NaCl-Lösung. Je nach Anwendung und Art der Organokklusion ist jedoch auch eine Befüllung mit einem Gas oder einem Gasgemisch wie Luft möglich. Die Befüllung erfolgt über den Ballonmund, der entweder von der Ballonhülle selbst oder - je nach Ausführungsvariante - durch das proximale Ende des Verlängerungselements gebildet wird.

Die Vorrichtung umfasst ferner einen separaten Hilfskatheter zur Platzierung des Ballonkörpers in dem zu behandelnden Organabschnitt und/oder zur Inflation des Ballonkörpers mit dem Fluid. Insbesondere bei der fetalchirurgischen Entfernung sowie im Falle einer ungeplanten EXIT-Prozedur stellt die Entfernung des Ballons ein erhebliches Problem dar, was nicht zuletzt auch zu der noch hohen Morbiditätsrate bei den Föten oder den Neugeborenen beiträgt. Erfindungsgemäß ist daher vorgesehen, dass der Ballonkörper mit einem flexiblen länglichen Verlängerungselement verbunden ist, welches sich schwanzartig von dem Ballonkörper, d.h. der Ballonhülle oder dem Ballonmund, filamentförmig erstreckt. Das mit dem Ballonkörper verbundene flexible längliche Verlängerungselement ist in seiner Längserstreckung gegenüber der Länge des Ballonkörpers um ein Vielfaches, d.h. um ein 2-, 3-, 4-, 5-, 6-, 7-, 8-, 9-, 10-faches oder mehr, verlängert.

Unter einem "flexiblen Verlängerungselement" im Sinne der Erfindung versteht man einen länglichen Körper mit einem Seitenverhältnis von Länge:Breite von wenigstens 4:1, vorzugsweise wenigstens 10:1 oder mehr. Flexibel bedeutet, dass das Verlängerungselement bei Bedarf manuell aufwickelbar, frei führbar und (im Gegensatz beispielsweise zu einem Draht) nicht starr ist. Bevorzugt besteht das flexible längliche Verlängerungselement aus Vollmaterial oder ist zumindest abschnittsweise innen hohl. Vorzugsweise handelt es sich dabei um ein Filament, einen Faden, eine Faser, ein Fasergemisch, eine Schnur, einen Schlauch, ein Garn oder ein Band. Bei einer Variante aus Vollmaterial ist das Verlängerungselement nicht mit dem Lumen des Ballonkörpers in fluider Verbindung. Alternativ kann das Verlängerungselement zumindest abschnittsweise einen Kanal umfassen, der beispielsweise zur Führung des Hilfskatheters für die Befüllung des Ballons dient. Der Kanal kann sich aber auch über die gesamte Länge des Verlängerungselements erstrecken.

In einer weiteren bevorzugten Variante ist das flexible längliche Verlängerungselement kraftschlüssig mit dem Ballonmund oder mit der Ballonhülle verbunden. So kann das Verlängerungselement von dem Ballonmund oder der Ballonhülle als Fortsatz fadenartig fortgeführt sein. Erfindungsgemäß verschließt das flexible Verlängerungselement den Ballonmund, beispielsweise indem ein Faden um den Ballonmund gewickelt ist, oder indem in einem zumindest abschnittsweisen hohlen Verlängerungselement eine separate Verschlusseinrichtung für den Ballonmund vorgesehen ist.

Der Ballonmund selbst kann auch durch die Ballonhülle oder durch ein in dem Ballonkörper eingesetztes Schlauchstück gebildet werden. In dem Ballonmund ist vorzugsweise eine Ventilklappe integriert. Bei der Ventilklappe handelt es sich beispielsweise um ein Plättchen oder eine fluiddichte Membran, das / die den Kanal fluiddicht verschließt.

Wenn das Verlängerungselement einen Kanal umfasst, der mit dem Lumen des Ballonkörpers in fluider Verbindung steht, dann ist die Ventilklappe in dem Kanal integriert und kann bei Einführung bzw. beim Herausziehen des Hilfskatheters den Kanal freigeben oder verschließen. Die Ventilklappe befindet sich vorzugsweise am distalen Ende des in dem Verlängerungselement verlaufenden Kanals, also an dem Ende, an dem der Hilfskatheter eingeführt wird.

Sofern das Verlängerungselement über die gesamte Länge als Schlauch mit einem darin verlaufenden Kanal oder Hohlraum ausgebildet ist, kann sich die Ventilklappe auch am distalen Ende des Verlängerungselements befinden. Bei dieser Variante wäre ausreichend, wenn die Klappe den Kanal nach Befüllung des Ballons fluiddicht verschließt. Eine Deflation des Ballons ist möglich, wenn das distale Ende des Verlängerungselements hinter der Ventilklappe (d.h. ballonseitig) abgeschnitten wird, was zur einem sofortigen Austritt von Fluid aus dem Ballonkörper führt und den chirurgischen Eingriff erheblich vereinfacht. Vorzugsweise ist die Ventilklappe aus demselben Material hergestellt wie das Verlängerungselement.

Erfindungsgemäß ist vorgesehen, dass sich der Ballonmund oder ein anderer Teil des Ballonsystems öffnet, wenn an dem Verlängerungselement mit einer bestimmten Kraft > 0.5 N, vorzugsweise einer Kraft zwischen 0.5 und 10 N, gezogen wird. Dies hat bei einer intrauterinen Trachealokklusion einen erheblichen Vorteil: so ist bekannt, dass der Fötus bereits in der Gebärmutter ein ausgeprägtes Spielverhalten entwickelt, so dass dieser ggf. selbst die vorgenommene Trachealokklusion am Ende der Schwangerschaft entfernen kann, indem der Ballonmund über das fadenartige flexible Verlängerungselement geöffnet wird. Der Ballon würde dann durch das Ausströmen des Fluids deflatiert. Ein Öffnen ist somit erst dann möglich, wenn der Fötus die für die Öffnung des Ballons erforderliche Kraft ausgebildet hat. Ferner ließe sich dadurch ein unbeabsichtigtes Öffnen der Klappe und damit eine Deflation des Ballons vermeiden.

Bei einer intrauterinen Trachealokklusion wird der Ballon in einem bestimmten Entwicklungsstadium in die Trachea des Fötus eingesetzt. Mit fortschreitender Schwangerschaft erweitert sich die Trachea im Zuge der Entwicklung des Kindes, so dass die Entfernung des Ballons leichter fällt. Das Entleeren des Ballons kann entweder durch Öffnen der Ventilklappe oder durch ein Aufreißen des Ballonmundes oder des Ballonkörpers über das Verlängerungselement erfolgen.

Der erfindungsgemäße Ballon bietet die Möglichkeit einer erleichterten Entfernung durch den Operateur oder durch den Patienten selbst, was von der jeweiligen Organokklusion abhängt. Für die Entfernung ist vorzugsweise vorgesehen, dass sich der Ballonmund oder der Ballonkörper selbst öffnet. Hierfür ist in einer bevorzugten Variante eine Sollbruchstelle im Ballonmund oder am Ballonkörper vorgesehen, die aufreißt, wenn an dem Verlängerungselement eine Ziehkraft ausgeübt wird. So kann vorgesehen sein, dass durch die Anwendung einer Kraft von > 0,5 N die Ballonhülle oder der Ballonmund an der Sollbruchstelle aufreißt.

In einer alternativen Ausführungsvariante umfasst das erfindungsgemäße Verlängerungselement eine Verschlussklappe am distalen Ende. Zur Entfernung des Ballons wird die Klappe abgeschnitten, um das Fluid oder die Flüssigkeit aus dem Ballon über den Katheter abzulassen. Danach wird der Ballon durch das Ziehen aus der Trachea entfernt.

Im Zuge einer fetoskopischen Entfernung bietet das Verlängerungselement gegenüber einem herkömmlichen Ballon erhebliche Vorteile, da das Verlängerungselement und dessen Lage sehr leicht lokalisiert und entfernt werden kann. Dadurch werden die Operationszeit und die damit einhergehenden fetalchirurgischen Risiken erheblich verringert.

Je nach Indikation und Art der durchzuführenden Organokklusion können die Ausmessungen des Ballonkörpers bzw. des fadenartigen flexiblen Verlängerungselements unterschiedlich sein. Vorzugsweise umfasst der Ballonkörper ein Volumen zwischen 0,25 und 9 ml, vorzugsweise zwischen 1 und 3 ml. Der Öffnungsdurchmesser des Ballonmundes variiert vorzugsweise zwischen 1,0 und 6,0 mm, vorzugsweise zwischen 1,5 und 3,0 mm. Der Ballonkörper weist in seiner Breite (B) und seiner Längserstreckung (L) vorzugsweise ein Seitenverhältnis B:L zwischen 7 x 10 mm und 70 x 120 mm, vorzugsweise zwischen 7 x 10 und 15 x 30 mm auf.

Der Ballon wird über die Einführung eines separaten Hilfskatheters mit dem Fluid entfaltet und das Fluid in dem Lumen des Ballonkörpers durch die verschließende fluiddichte Klappe gehalten. Durch ein Ziehen an dem Verlängerungselement kann sich die Ventilklappe und somit der Ballonmund öffnen, so dass das Fluid aus dem Lumen des Ballonkörpers entweichen kann. In einer bevorzugten Ausführungsform ist das Verlängerungselement direkt mit der Ventilklappe in dem Ballonmund kraftschlüssig verbunden. In einer alternativen Ausführungsvariante kann die Ventilklappe durch den Operateur oder den Patienten selbst über das Verlängerungselement betätigt werden, beispielsweise durch Anwendung einer Ziehkraft.

Die Ballonhülle des Ballonkörpers selbst besteht vorzugsweise aus einem Polyurethan, einer Polyurethan-Polyvinylchloridmischung oder aus Latex. Vorzugsweise handelt es sich bei dem fadenartigen flexiblen Verlängerungselement um einen mit dem Ballonmund verbundenen Polypropylenfaden.

Die vorliegende Erfindung betrifft auch die Verwendung der oben beschriebenen Vorrichtung für die Herstellung eines Medizinproduktes für den Einsatz bei Organokklusionen bei Menschen oder Säugetieren, vorzugsweise bei einer intrauterinen Trachealokklusion bei der Vorbeugung und Behandlung einer angeborenen fetalen Zwerchfellhernie. Wie beschrieben, stellt die intrauterine fetale Trachealokklusion (FETO) eine pränatale Therapieoption bei der Behandlung einer kongenitalen Zwerchfellhernie mit schlechter Prognose dar, wobei als häufigste Komplikation ein iatrogener Blasensprung mit den damit verbundenen Risiken auftritt. Durch die Verwendung eines Ballons mit einem daran ausgebildeten fadenartigen flexiblen Verlängerungselement lässt sich das Risiko durch eine Minimierung der amnialen Verletzung und ggf. auch durch eine Reduzierung der notwendigen Eingriffe drastisch verringern. Die erfindungsgemäße Vorrichtung stellt eine erhebliche Verbesserung bei einer intrauterinen Trachealokklusion dar, da bei einer Minimierung der Therapierisiken die Indikation zur Operation im Rahmen einer Prädiktion des Outcomes großzügiger gestellt werden kann und so weitaus mehr Kinder von der fetalchirurgischen Therapie profitieren können. Durch den Einsatz des erfindungsgemäßen Ballons im Zusammenhang mit einer FETO wird ein deutlich besseres Ergebnis ("Outcome") erzielt als mit einem konservativen Management.

Die erfindungsgemäße Vorrichtung eignet sich jedoch nicht nur für eine fetalchirurgische Therapie, beispielsweise im Rahmen einer Trachealokklusion bei der Behandlung oder Vorbeugung einer angeborenen fetalen Zwerchfellhernie, sondern auch für eine Reihe weiterer Indikationen, die eine Organokklusion mit Hilfe eines Ballonkörpers vorsehen. Hierzu gehört beispielsweise eine Okklusion der oberen Luftwege, beispielsweise im Rahmen der Blutstillung, wie sie bei Nasenblutungen oder bei Blutungen aus dem urogenitalen Trakt auftritt.

Solche Blutungen können durch das Einführen eines entfalteten Ballons in den betreffenden Organabschnitt gelindert bzw. verhindert werden, d.h. das erfindungsgemäße Ballonsystem kann auch erfolgreich eine klassische Tamponade ersetzen. Solche Blutungen können jedoch auch durch Traumaverletzungen wie beispielsweise Prellungen oder Schürfwunden aus der Nasenschleimhaut auftreten oder durch Geschwüre, Varizen, Polypen oder Entzündungen (z.B. Rhinitis). Daneben können auch allgemeine Stresssituationen zu Blutungen führen wie beispielsweise Hämophilie, bestimmte Formen der Anämie, Bluthochdruck, Nieren- und Lebererkrankungen, Leukämien, Skorbut, Infektionskrankheiten etc. Der erfindungsgemäße, mit einem fadenartigen flexiblen Verlängerungselement bestückte Ballonkörper eignet sich somit allgemein als Medizinprodukt bei der Vorbeugung oder Behandlung von Blutungen durch Organokklusionen, beispielsweise Blutungen des Ösophagus oder der Gehörgänge. Die erfindungsgemäße Vorrichtung kann im Rahmen einer Palliativmedizin bei Blutungen aus dem urogenitalen Trakt verwendet werden.

Daneben ist die erfindungsgemäße Vorrichtung auch einsetzbar für langsame Dilationen bei einer Organverengung, bei Vernarbungen, beispielsweise des Ösophagus, der Urethra, der Vagina etc. Insofern eignet sich die erfindungsgemäße Vorrichtung auch zum Einsatz im Rahmen eines plastisch-chirurgischen Eingriffes oder einer plastischen Rekonstruktion.

Aufgrund der hohen Morbidität und der mit einer fetoskopischen Positionierung eines Trachealballons einhergehenden Komplikation eines iatrogenen Blasensprungs ist jedoch der Einsatz der erfindungsgemäßen Vorrichtung als Medizinprodukt im Rahmen einer intrauterinen fetalen Trachealokklusion ein bevorzugtes Anwendungsgebiet, da hier die Morbiditätsraten der betroffenen Patienten erheblich gesenkt werden können, indem ein Auffinden und/oder eine Entfernung des Trachealballons gegenüber der bisherigen Variante erheblich verbessert werden kann. Die Verwendung eines fadenartigen flexiblen länglichen Verlängerungselements ermöglicht eine fetoskopische Entfernung, wie es bislang nicht möglich war. Die Operationszeiten und die damit verbundenen chirurgischen Eingriffe werden erheblich verringert, wodurch sich auch das Morbiditätsrisiko und mögliche Komplikationen erheblich verringern lassen. In einer bevorzugten Variante können der Ballonkörper bzw. die Ballonhülle sowie auch das Verlängerungselement aus einem bio-abbaubaren Material bestehen, so dass bei einer intrakorporalen Ballonentfernung zunächst eine Deflation des Ballons über das Verlängerungselement und ein anschließender Abbau des Ballonmaterials bzw. des Verlängerungselements erfolgt.

Die Erfindung wird in den nachfolgenden Ausführungsbeispielen näher erläutert, wobei in den Zeichnungen unterschiedliche Varianten des erfindungsgemäßen Ballons gezeigt sind.

### Figuren:

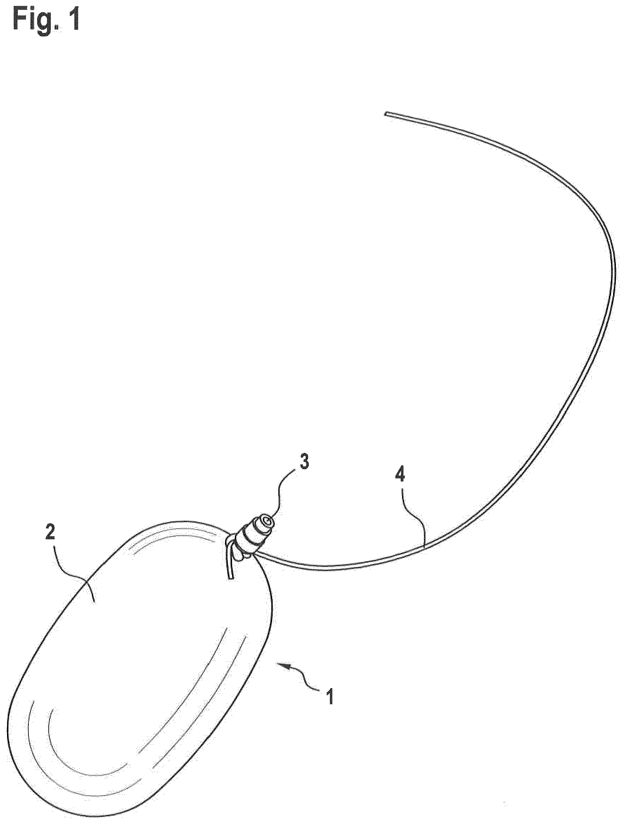

In Fig. 1 ist eine erste Ausführungsvariante der erfindungsgemäßen Vorrichtung (ohne Hilfskatheter) gezeigt, bestehend aus einem Ballonkörper 1, einer Ballonhülle 2 und einem Ballonmund 3. An dem Ballonmund 3 befindet sich ein fadenartiges flexibles Verlängerungselement 4 in Form eines Polypropylenfadens, der um den Ballonmund 3 gewickelt ist. Das Verlängerungselement 4 ist in der gezeigten Variante nicht mit dem Lumen des Ballonkörpers verbunden und innen nicht hohl.

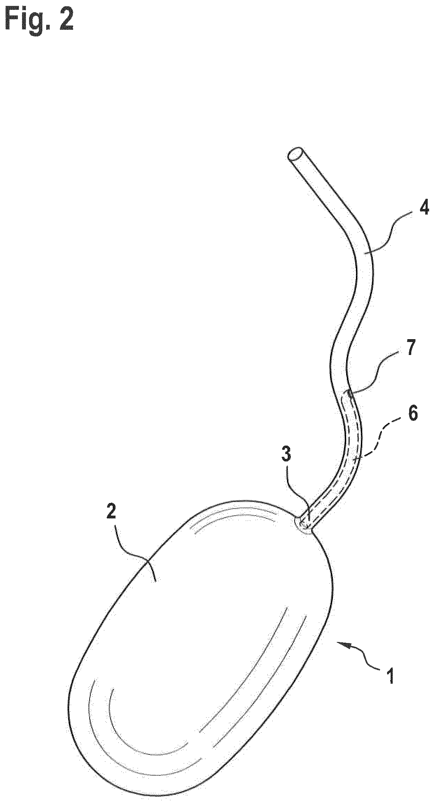

In Fig. 2 ist eine weitere Ausführungsvariante gezeigt, bei der innerhalb des flexiblen länglichen Verlängerungselements 4 an dessen proximalen Ende abschnittsweise ein Kanal 6 verläuft, der mit dem Lumen des Ballonkörpers 1 in fluider Verbindung steht. Am distalen Ende des Kanals 6 ist eine Verschlusseinrichtung vorgesehen, um einen Austritt von Fluid aus dem Ballon zu vermeiden. Die Öffnung 7 des Kanals 6 am distalen Ende dient ferner der Einführung des Hilfskatheters zur Positionierung und/oder Befüllung des Ballons.

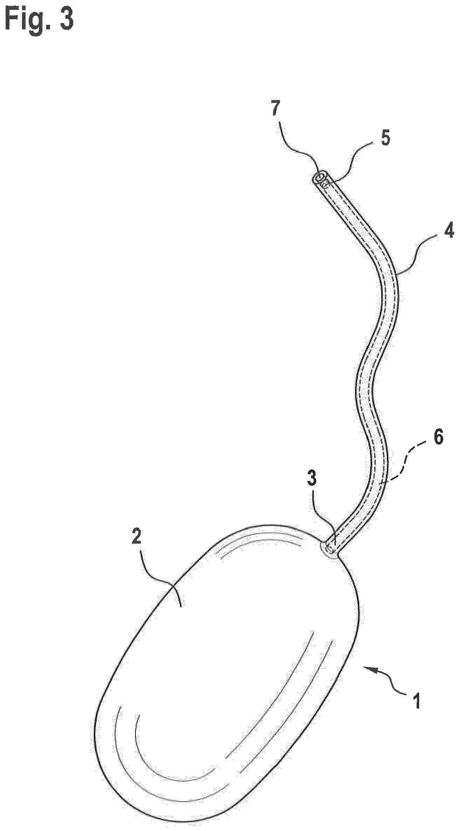

In Fig. 3 ist eine weitere Ausführungsvariante gezeigt, bei der in dem Verlängerungselement 4 ein Kanal 6 über die gesamte Länge des Verlängerungselements 4 verläuft. Am distalen Ende des Verlängerungselements ist in dem Kanal 6 eine Ventilklappe 5 angeordnet. Das Entleeren des Ballons erfolgt beispielsweise durch Abschneiden des distalen Endes des Verlängerungselements 4 hinter der Ventilklappe 5.

### Klinische Studie:

Im Rahmen einer Studie wurden Patienten mit einer sonographisch diagnostizierten, ausgeprägten fetalen Zwerchfellhernie mit einem o/e LV-Verhältnis von 15% ("observed/expected lung volume ratio", MRT-Befund) einer Behandlung mit der erfindungsgemäßen Vorrichtung unterzogen. Ein mit einem Polypropylenfaden bestückter Ballon gemäß der Figur 1 wurde zur Durchführung einer intrauterinen Trachealokklusion angewendet. Der Ballonkörper 1 bestand aus einem Latexballon mit einem Volumen von 2,5 ml und einer Größe von 12 x 28 mm. An dem Ballonmund 3 wurde ein Verlängerungselement 4 in Form eines Polypropylenfadens (monophil 2-0 Polypropylen, 5/0) mit einer Länge von 7 cm befestigt. Üblicherweise erfolgt der Verschluss der fetalen Trachea intrauterin zwischen der 26. und 28. Schwangerschaftswoche (SSW), wodurch der erhöhte hydrostatische Druck im Bronchialsystem ansteigt. Die Entfernung des intratrachealen Ballons erfolgte im Rahmen eines zweiten fetalchirurgischen Eingriffs zwischen der 34. und 35. SSW. Anschließend erfolgte eine fetalchirurgische Behandlung nach der 28 + 4 Voll. SSW. Der Eingriff erfolgte mit Hilfe eines 1,2 mm Fetoskopes unter 3-D-Ultraschallkontrolle.

Der Fötus wurde problemlos bis vor die Bifurcatio trachae intubiert. Die Optik wurde vorübergehend entfernt und der Ballon mit Hilfe eines Hilfskatheters unter Ultraschallkontrolle in die fetale Trachea eingebracht (Tchirikov M, Gatopoulos G, Strohner M, Puhl A, Steetskamp J. Two new approaches in intrauterine tracheal occlusion using an ultrathin fetoscope. Laryngoscope. 2010 Feb;120(2):394-8). Nachdem der Ballon mit dem fadenartigen flexiblen Verlängerungselement 4 über der Bifurkation platziert wurde, erfolgte die Entfaltung des Ballonkörpers 1 mit 2,5 ml NaCI-Lösung. Während dieses Vorganges wurden die Lokalisation des mit dem Verlängerungselement 4 bestückten Ballons und der Zustand des Fötus sonografisch kontrolliert. Anschließend wurde der Hilfskatheter entfernt und durch die Optik ersetzt. Sonografische Darstellungen des Ballons in situ zeigen, dass sich der Trachealballon mit dem Verlängerungselement 4 an der gewünschten Position befand.

Im weiteren Verlauf vergrößerte sich das observed/expected-LV-Verhältnis von 15% auf 134%. Zwei Monate nach der Einlage erfolgte die Entfernung des Trachealballons. Dabei konnte der Polyproplyenfaden bei der fetoskopischen Entfernung leicht lokalisiert werden. Die anschließende Entbindung erfolgte per sectionem ("Kaiserschnitt"). Die angeborene Zwerchfellhernie wurde am folgenden Tag erfolgreich operiert. Nach einem Zeitraum von einem Jahr zeigte das Kind bei der Untersuchung keine bleibenden Schäden.

Durch die Verbindung des Ballonkörpers 1 mit einem fadenartigen flexiblen Verlängerungselement 4, beispielsweise einem Polypropylenfaden, lassen sich das Auffinden sowie die Entfernung des Trachealballons deutlich vereinfachen. Die Operationszeit konnte hierdurch im Vergleich zur klassischen Methode deutlich verringert werden.

Die Verbindung des Ballons mit einem Verlängerungselement 4 bietet ferner eine intrauterine Möglichkeit zur Ballonentfernung durch den Fötus selbst. Bei dieser Ausführungsvariante kann auf einen zweiten fetoskopischen chirurgischen Eingriff in der 34. SSW gänzlich verzichtet werden. Die erfindungsgemäße Vorrichtung bietet somit auch eine Verlängerung der Wirkungsdauer bei einer Trachealokklusion auf das Wachstum der Lunge bis zum Entbindungszeitpunkt. Doch auch bei anderen Indikationen, welche eine Organokklusion vorsehen, beispielsweise eine Okklusion der oberen Luftwege zur Blutstillung oder von anderen Organen bei Organblutungen, kann der Patient den Ballonkörper 1 durch ein Ziehen an dem Verlängerungselement 4 aus dem betroffenen Behandlungsabschnitt oder dem Organ selbst entfernen. Somit ist eine Entfernung des Ballons bei diesen Indikationen weitaus einfacher, als es bei herkömmlichen Ballons der Fall war.

## Patentansprüche

1. Vorrichtung zur Verwendung bei Organokklusionen bei Menschen oder Säugetieren, insbesondere für die Durchführung einer intrauterinen Trachealokklusion bei der Behandlung einer angeborenen fetalen Zwerchfellhernie, umfassend
- einen mit einem Fluid befüllbaren Ballonkörper (1) mit einer Ballonhülle (2), die ein Lumen zur Aufnahme des Fluids bildet und einem Ballonmund (3) zur Einführung des Fluids in das Lumen,
- einen separaten Hilfskatheter zur Platzierung des Ballonkörpers (1) in den zu behandelnden Organabschnitt und/oder zur Inflation des Ballonkörpers (1) mit dem Fluid,
wobei der Ballonkörper (1) mit einem flexiblen länglichen Verlängerungselement (4) verbunden ist, das den Ballonmund (3) oder einen anderen Teil des Ballonsystems bei Anwendung einer Ziehkraft von > 0.5 N öffnet, **dadurch gekennzeichnet, dass** das längliche Verlängerungselement (4) den Ballonmund (3) verschließt.

2. Vorrichtung nach Anspruch 1, **dadurch gekennzeichnet, dass** es sich bei dem flexiblen länglichen Verlängerungselement (4) um ein Filament, einen Faden, eine Faser, ein Fasergemisch, eine Schnur, einen Schlauch, ein Garn oder ein Band handelt.

3. Vorrichtung nach Anspruch 1 oder 2, **dadurch gekennzeichnet, dass** das flexible längliche Verlängerungselement (4) kraftschlüssig mit dem Ballonmund (3) oder mit der Ballonhülle (2) verbunden ist.

4. Vorrichtung nach Anspruch 1, **dadurch gekennzeichnet, dass** in dem Ballonmund (3) eine Ventilklappe (5) integriert ist.

5. Vorrichtung nach Anspruch 4, **dadurch gekennzeichnet, dass** die in dem Ballonmund (3) integrierte Ventilklappe (5) mit dem flexiblen länglichen Verlängerungselement (4) kraftschlüssig zur Deflation des Ballons verbunden ist.

6. Vorrichtung nach einem der vorhergehenden Ansprüche, **dadurch gekennzeichnet, dass** der Ballonkörper (1) ein Volumen zwischen 0,25 und 9 ml, vorzugsweise zwischen 1 und 3 ml, umfasst und/oder dass der Ballonmund einen Öffnungsdurchmesser zwischen 1,0 und 6,0 mm, vorzugsweise zwischen 1,5 und 3,0 mm, aufweist, und/oder dass der Ballonkörper (1) in seiner Breite und Längserstreckung ein Seitenverhältnis zwischen 7 x 10 mm und 70 x 120 mm aufweist.

7. Vorrichtung nach einem der vorhergehenden Ansprüche, **dadurch gekennzeichnet, dass** das flexible längliche Verlängerungselement (4) in seiner Längserstreckung wenigstens ein Vielfaches der Länge des Ballonkörpers (1) entspricht und das Verlängerungselement (4) entweder aus Vollmaterial besteht oder zumindest abschnittsweise innen einen Kanal (6) umfasst.

## Claims

1. Device for use in organ occlusions in humans or mammals, in particular for carrying out an intrauterine tracheal occlusion in the course of treating a foetal congenital diaphragmatic hernia, comprising
- a balloon body (1) that can be filled with a fluid and has a balloon shell (2) which forms a lumen for receiving the fluid and a balloon mouth (3) for introducing the fluid into the lumen,
- a separate auxiliary catheter for placing the balloon body (1) into the organ portion to be treated and/or for inflating the balloon body (1) with the fluid,
the balloon body (1) being connected to a flexible elongate extension element (4) that opens the balloon mouth (3) or another part of the balloon system by applying a drawing force of >0.5 N, **characterised in that** the elongate extension element (4) closes the balloon mouth (3).

2. Device according to claim 1, **characterised in that** the flexible elongate extension element (4) is a filament, a thread, a fibre, a fibre blend, a cord, a tube, a yarn or a ribbon.

3. Device according to either claim 1 or claim 2, **characterised in that** the flexible elongate extension element (4) is frictionally connected to the balloon mouth (3) or to the balloon shell (2).

4. Device according to claim 1, **characterised in that** a valve flap (5) is integrated in the balloon mouth (3).

5. Device according to claim 4, **characterised in that** the valve flap (5) integrated in the balloon mouth (3) is frictionally connected to the flexible elongate extension element (4) in order to deflate the balloon.

6. Device according to any of the preceding claims, **characterised in that** the balloon body (1) has a volume between 0.25 and 9 ml, preferably between 1 and 3 ml, and/or **in that** the balloon mouth has an opening diameter between 1.0 and 6.0 mm, preferably between 1.5 and 3.0 mm, and/or **in that** the balloon body (1) has a side ratio between 7×10 mm and 70×120 mm with regard to the width and longitudinal extension thereof.

7. Device according to any of the preceding claims, **characterised in that** the longitudinal extension of the flexible elongate extension element (4) corresponds to at least one multiple of the length of the balloon body (1), and the extension element (4) either consists of solid material or comprises an internal channel (6), at least in portions.

## Revendications

1. Dispositif pour l'utilisation dans le cas d'occlusions d'organes chez l'homme ou les mammifères, en particulier pour effectuer une occlusion trachéale intra-utérine lors du traitement d'une hernie diaphragmatique foetale congénitale, comprenant
- un corps de ballonnet (1) pouvant être rempli d'un fluide, avec une enveloppe de ballonnet (2) qui forme une lumière pour recevoir le fluide et une embouchure de ballonnet (3) pour introduire le fluide dans la lumière,
- un cathéter auxiliaire séparé pour placer le corps de ballonnet (1) dans la portion de l'organe à traiter et/ou pour gonfler le corps de ballonnet (1) avec le fluide,
le corps de ballonnet (1) étant connecté à un élément de prolongement allongé flexible (4) qui ouvre l'embouchure de ballonnet (3) ou une autre partie du système de ballonnet lors de l'utilisation d'une force de traction supérieure à 0,5 N,
**caractérisé en ce que** l'élément de prolongement allongé (4) ferme l'embouchure de ballonnet (3).

2. Dispositif selon la revendication 1, **caractérisé en ce que** l'élément de prolongement allongé flexible (4) est un filament, un fil, une fibre, un mélange de fibres, un cordon, un tuyau souple, un filin ou une bande.

3. Dispositif selon la revendication 1 ou 2, **caractérisé en ce que** l'élément de prolongement allongé flexible (4) est connecté par engagement par force avec l'embouchure de ballonnet (3) ou avec l'enveloppe de ballonnet (2).

4. Dispositif selon la revendication 1, **caractérisé en ce qu'**un clapet de valve (5) est intégré dans l'embouchure de ballonnet (3).

5. Dispositif selon la revendication 4, **caractérisé en ce que** le clapet de valve (5) intégré dans l'embouchure de ballonnet (3) est connecté par engagement par force avec l'élément de prolongement allongé flexible (4) afin de dégonfler le ballonnet.

6. Dispositif selon l'une quelconque des revendications précédentes, **caractérisé en ce que** le corps de ballonnet (1) comprend un volume compris entre 0,25 et 9 ml, de préférence entre 1 et 3 ml, et/ou **en ce que** l'embouchure de ballonnet présente un diamètre d'ouverture compris entre 1,0 et 6,0 mm, de préférence entre 1,5 et 3,0 mm, et/ou **en ce que** le corps de ballonnet (1) présente un rapport latéral entre sa largeur et son étendue longitudinale compris entre 7 x 10 mm et 70 x 120 mm.

7. Dispositif selon l'une quelconque des revendications précédentes, **caractérisé en ce que** l'élément de prolongement allongé flexible (4) correspond, dans son étendue longitudinale, à au moins un multiple de la longueur du corps de ballonnet (1) et l'élément de prolongement (4) se compose soit d'un matériau massif soit comprend au moins en partie un canal (6) à l'intérieur.
